# EUROPEAN PATENT SPECIFICATION

(11) **EP 4 340 926 B1**
(45) Date of publication and mention of the grant of the patent: **24.06.2026**
(21) Application number: 21734982.8
(22) Date of filing: 20.05.2021
(51) Int. Cl.: A61M 39/02, A61M 39/04

(54) **LOW-PROFILE TRANSITIONABLE ACCESS PORT**
NIEDRIGPROFILIGER, ÜBERGANGSFÄHIGER ZUGANGSPORT
PORT D'ACCÈS TRANSITIONNEL À PROFIL BAS

(43) Date of publication of application: 27.03.2024
(73) Proprietor: Bard Peripheral Vascular, Inc., Franklin Lakes, NJ 07417 (US)
(72) Inventor: HOYE, Jessica, Phoenix, AZ 85032 (US); DENSLEY, Bryon, Ray, Fremont, CA 94538 (US); ANDERSEN, Christian, Kaysville, UT 84037 (US); FIUMEFREDDO, Diana, Salt Lake City, UT 84121 (US); THOMAS, Ian, N., West Bountiful, UT 84087 (US)
(74) Representative: Hoffmann Eitle
(86) International application number: PCT/US2021/033386
(87) International publication number: WO 2022/245358

(56) References cited:
- WO-A1-2019/200304
- US-A1- 2011 137 288

## Description

### BACKGROUND

US 2011/0137288 A1 discloses an access port, wherein the access port may include a body having an exterior surface and a chamber defined thereon, a bore defined in the body providing fluid communication between the chamber and the exterior surface, a needle in fluid communication with the chamber and the exterior surface, a needle in fluid communication with the chamber, a passage defined in the body providing communication between the chamber and the exterior surface, a seal secured within the passage, and an actuator in communication with the needle, configured to move the needle relative to the passage or move the passage relative to the needle.

WO 2019/200304 A1 discloses a low profile single and dual vascular access device.

### SUMMARY

The claimed subcutaneous access port and method of manufacturing a subcutaneous access port are defined by appended claims 1 and 9 respectively. Briefly summarized, embodiments disclosed herein are directed to a subcutaneous access port transitionable between an expanded configuration and a collapsed configuration, and associated methods thereof. The port includes a body having a lower housing and an upper housing slidably engaged therewith. The lower housing and the upper housing co-operate to define a reservoir. In the expanded configuration the port defines a first port volume and a first reservoir volume. In the collapsed configuration the port defines a second port volume that is less than the first port volume, and a second reservoir volume that is less than the first reservoir volume. Advantageously, the port can transition to the collapsed configuration to provide a reduced overall size or outer profile for insertion. The port provides a reduced overall size between access events. As such, the port can require a smaller incision site, require fewer stitches or no stitches at all, improving patient recovery times, patient comfort, reducing scarring and improving aesthetics.

Disclosed herein is a subcutaneous access port including, a body having a lower housing and an upper housing slidably engaged therewith and co-operating to define a reservoir, the body transitionable between an expanded configuration and a collapsed configuration, the collapsed configuration defining a smaller overall profile, a needle penetrable septum disposed in a reservoir opening disposed in a top surface of the upper housing and configured to provide percutaneous access thereto, and a port stem defining a stem lumen in fluid communication with the reservoir. The reservoir in the expanded configuration defines a first reservoir volume, and the reservoir in the collapsed configuration defines a second reservoir volume, the second reservoir volume being less than the first reservoir volume

In some embodiments, the port body in the expanded configuration defines a first port height, and wherein the port body in the collapsed configuration defines a second port height, the second port height being less than the first port height. In some embodiments, the port body in the expanded configuration defines a first port volume, and the port body in the collapsed configuration defines a second port volume, the second port volume being less than the first port volume. In some embodiments, the reservoir in the expanded configuration defines a first reservoir height, and the reservoir in the collapsed configuration defines a second reservoir height, the second reservoir height being less than the first reservoir height.

In some embodiments, the second reservoir volume defines a zero volume or *a de minimis* volume. In some embodiments, the lower housing is telescopically engaged with the upper housing and slidably engaged to transition between the expanded configuration and the collapsed configuration. In some embodiments, an outer surface of the upper housing is slidably engaged with an inner surface of the lower housing. In some embodiments, an inner surface of the upper housing is slidably engaged with an outer surface of the lower housing. In some embodiments, one of the upper housing or the septum occludes the stem lumen when the port is in the collapsed configuration.

In some embodiments, the subcutaneous access port further includes a biasing member configured to bias the port towards the expanded configuration. In some embodiments, the subcutaneous access port further includes a latching mechanism transitionable between a locked position and an unlocked position, and configured to retain the upper housing in one of the expanded configuration or the collapsed configuration. In some embodiments, the latching mechanism is configured to lock the upper housing in an expanded configuration when a needle accesses the reservoir. In some embodiments, the latching mechanism is configured to engage and retain the upper housing in a collapsed configuration and disengage the upper housing when a top surface of the port is depressed.

In some embodiments, the subcutaneous access port further includes an actuator coupled to the latching mechanism and configured to transition the latching mechanism between a locked position and an unlocked position. In some embodiments, the actuator is one of a push button, slider, or rocker switch disposed on one of a top surface or a side surface of the body. In some embodiments, the actuator includes a magnetic switch configured to transition the latching mechanism between a locked position and an unlocked position when a magnetic having a magnetic field is placed proximate the actuator. In some embodiments, the actuator is configured to receive a signal to transition the latching mechanism between a locked position and an unlocked position, the signal including one of a magnetic, electro-magnetic, radio-frequency, acoustic, or optical modality signal.

Also disclosed is a method of manufacturing a subcutaneous access port including, forming a port body having a lower housing and an upper housing slidably engaged therewith and co-operating to define a reservoir, the body transitionable between an expanded configuration and a collapsed configuration, the collapsed configuration defining a smaller overall profile, coupling a needle penetrable septum disposed in a reservoir opening disposed in a top surface of the upper housing and configured to provide percutaneous access thereto, and forming a port stem coupled to the body and defining a stem lumen in fluid communication with the reservoir. The reservoir in the expanded configuration defines a first reservoir volume, and the reservoir in the collapsed configuration defines a second reservoir volume, the second reservoir volume being less than the first reservoir volume.

In some embodiments, the port body in the expanded configuration defines a first port height, and wherein the port body in the collapsed configuration defines a second port height, the second port height being less than the first port height. In some embodiments, the port body in the expanded configuration defines a first port volume, and the port body in the collapsed configuration defines a second port volume, the second port volume being less than the first port volume. In some embodiments, the reservoir in the expanded configuration defines a first reservoir height, and the reservoir in the collapsed configuration defines a second reservoir height, the second reservoir height being less than the first reservoir height.

In some embodiments, the second reservoir volume defines a zero volume or a *de minimis* volume. In
some embodiments, the method further includes telescopically engaging the lower housing with the upper housing to slidably transition between the expanded configuration and the collapsed configuration. In some embodiments, an outer surface of the upper housing is slidably engaged with an inner surface of the lower housing.

In some embodiments, an inner surface of the upper housing is slidably engaged with an outer surface of the lower housing. In some embodiments, one of the upper housing or the septum occludes the stem lumen when the port is in the collapsed configuration. In some embodiments, the method further includes forming a biasing member within the port body configured to bias the port towards the expanded configuration. In some embodiments, the method further includes a latching mechanism transitionable between a locked position and an unlocked position, and configured to retain the upper housing in one of the expanded configuration or the collapsed configuration.

In some embodiments, the latching mechanism is configured to lock the upper housing in an expanded configuration when a needle accesses the reservoir. In some embodiments, the latching mechanism is configured to engage and retain the upper housing in a collapsed configuration and disengage the upper housing when a top surface of the port is depressed. In some embodiments, the method further includes an actuator coupled to the latching mechanism and configured to transition the latching mechanism between a locked position and an unlocked position.

In some embodiments, the actuator is one of a push button, slider, or rocker switch disposed on one of a top surface or a side surface of the body. In some embodiments, the actuator includes a magnetic switch configured to transition the latching mechanism between a locked position and an unlocked position when a magnetic having a magnetic field is placed proximate the actuator. In some embodiments, the actuator is configured to receive a signal to transition the latching mechanism between a locked position and an unlocked position, the signal including one of a magnetic, electro-magnetic, radio-frequency, acoustic, or optical modality signal.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 shows a perspective view of a port coupled to a catheter, in accordance with embodiments disclosed herein.
FIG. 2A shows a longitudinal cross-section view of the port in an expanded configuration, in accordance with embodiments disclosed herein.
FIG. 2B shows a longitudinal cross-section view of the port in a collapsed configuration, in accordance with embodiments disclosed herein.
FIG. 3A shows a lateral cross-section view of the port in an expanded configuration, in accordance with embodiments disclosed herein.
FIG. 3B shows a lateral cross-section view of the port in a collapsed configuration, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

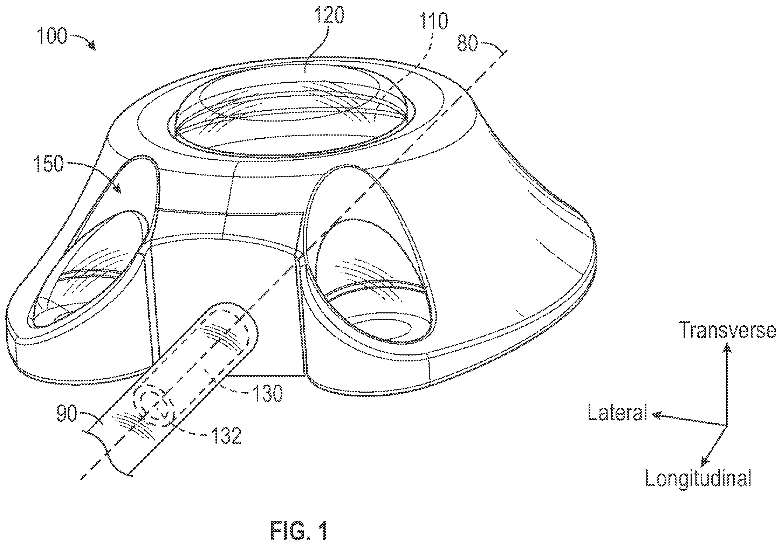

To assist in the description of embodiments described herein, as shown in FIG. 1, a longitudinal axis extends substantially parallel to an axial length of the port stem. A lateral axis extends normal to the longitudinal axis, and a transverse axis extends normal to both the longitudinal and lateral axes. As used herein, a horizontal plane extends along the lateral and longitudinal axes. A vertical plane extends normal to the horizontal plane.

Unless defined otherwise, all technical and scientific terms used herein have the same meaning as commonly understood by those of ordinary skill in the art.

FIG. 1 shows a vascular access device, or "port" 100 configured for subcutaneous placement. The port 100 includes a body 150 defining a reservoir 110 and including a needle penetrable septum 120 thereover. The port 100 further includes a port stem 130, defining a stem lumen 132, and is in fluid communication with the reservoir 110. The port stem 130 can engage a lumen of a catheter 90 in an interference fit, to provide fluid communication therewith. A distal tip of the catheter 90 can be disposed within the vasculature of a patient. In use, an access needle can penetrate the skin surface and access the reservoir by piercing the septum 120. Fluids can then be disposed within the reservoir 100, or withdrawn from the reservoir 110.

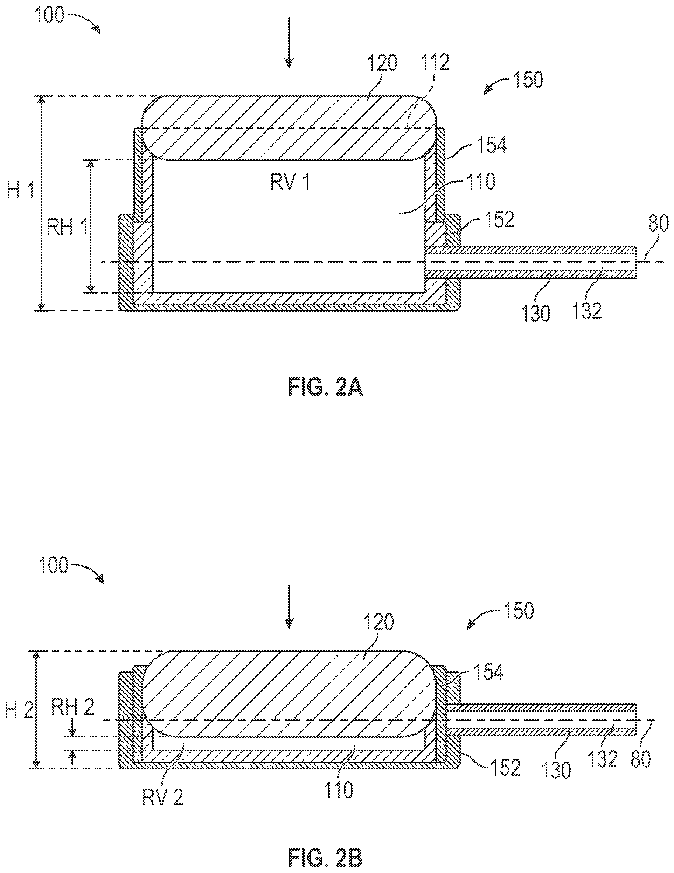

In an embodiment, as shown in FIGS. 2A-2B, the port 100 includes a port body 150 including a first housing, or lower housing 152, and a second housing, or an upper housing 154, slidably engaged therewith. The lower housing 152 and the upper housing 154 co-operate to define the reservoir 110. In an embodiment, the upper housing 154 defines a reservoir opening 112 disposed in a top surface thereof and configured to retain the needle-penetrable septum 120 therein. In an embodiment, the septum 120 can be formed of a silicone rubber or similar suitable material. In use, the reservoir 110 can be accessed percutaneously by an access needle, penetrating the septum 120 and fluidly accessing the reservoir 110.

In an embodiment, the port 100 further includes a port stem 130 extending along an axis 80. The stem 130 defines a stem lumen 132 that is in fluid communication with the reservoir 110. The stem 130 can be configured to be coupled to a catheter 90 or similar device, configured to access a vasculature of a patient. The catheter 90 can include an elongate body defining a lumen 92 extending therethrough. In an embodiment, the port body 150 can be formed of a substantially rigid material including plastic, polymer, metal, alloy, composite, or the like.

In an embodiment, the port 100 is transitionable between an expanded configuration (FIG. 2A) and a collapsed configuration (FIG. 2B) to facilitate subcutaneous placement. In an embodiment, the lower housing 152 and the upper housing 154 are slidably engaged with each other and can transition between a first, or expanded, configuration (FIG. 2A) and a second, or collapsed configuration (FIG. 2B). In an embodiment, an outer surface of the upper housing 154 can be telescopically engaged with an inner surface of the lower housing 152. In an embodiment, an inner surface of the upper housing 154 can be telescopically engaged with an outer surface of the lower housing 152.

In an embodiment, in the expanded configuration, the port 100 can define a first port height (*H1*)*.* In an embodiment, in the expanded configuration, the reservoir 110 can define a first reservoir height (*RH1*)*.* In an embodiment, in the collapsed configuration, the port 100 can define a second port height *(H2).* The second port height *(H2)* can be less than the first port height (*H1)*. In an embodiment, in the collapsed configuration, the reservoir 110 can define a second reservoir height (*RH1*)*.* The second reservoir height (*RH2*) being less than the first reservoir height (*RH1*)*.*

In an embodiment, in the expanded configuration, the port 100 can define a first port volume (*V1*)*.* In an embodiment, in the collapsed configuration, the port 100 can define a second port volume (*V2*). The second port volume (*V2*) being less than the first port volume (*V1*)*.* In an embodiment, in the expanded configuration, the reservoir 110 can define a first reservoir volume (*RV1*)*.* In an embodiment, in the collapsed configuration, the reservoir 110 can define a second reservoir volume (*RV2*)*.* The second reservoir volume (*RV2*) being less than the first reservoir volume (*RV1*)*.* In an embodiment, the second reservoir volume (*RV2*) can define a zero volume or a *de minimis* volume.

In an embodiment, a portion of the septum 120 or a portion of the body 150, e.g. a portion of the lower housing 152 or a portion of the upper housing 154, can occlude the port stem lumen 132 when the port 100 is in the collapsed configuration. As such, fluid communication between the reservoir 110 and the catheter lumen 92, coupled to the port stem 130 can be inhibited.

In an embodiment, the port 100 can include a biasing member (e.g. spring or the like) configured to bias the port towards the expanded configuration. In an embodiment, the port 100 can include a latch mechanism configured to selectively retain the port 100 in one of the expanded or collapsed configurations. In use, a clinician can selectively transition the port 100 between the expanded configuration and the collapsed configuration.

In an embodiment, as shown in FIGS. 2A-2B, a clinician can depress the top surface of the port 100, e.g. a top surface of the septum 120 or a top surface of the upper housing 154, to overcome the force of the biasing member and slidably transition the upper housing 154 from the expanded configuration (FIG. 2A) to the collapsed configuration (FIG. 2B). Once in the collapsed configuration, the latch mechanism can engage the upper housing 154 to retain the upper housing 154 in the collapsed configuration.

In an embodiment, the clinician can selectively transition the port 100 from the collapsed configuration to the expanded configuration. The clinician can depress the top surface of the port 100, e.g. a top surface of the septum 120 or a top surface of the upper housing 154, to overcome the force of the biasing member and disengage the latching mechanism. The clinician can then release the top surface of the port 100 and allow the biasing member to transition the upper housing 154 to the expanded configuration.

In an embodiment, the force of the biasing member can maintain the port 100 in the expanded configuration. In an embodiment, the force of the biasing member can maintain the port 100 in the expanded configuration even when the access needle is urged through the needle-penetrable septum 120 to access the reservoir 110 therebelow. In an embodiment, the latching mechanism can be configured to maintain the port 100 in the expanded configuration. As such, a clinician can urge the access needle through the septum 120 without depressing the top surface of the port 100 and transitioning the port from the expanded configuration to the collapsed configuration unintentionally.

In an embodiment, the latching mechanism can be transitioned between a locked position and an unlocked position. In the locked position the latching mechanism is prevented from disengaging the upper housing 154. In the unlocked position, the latching mechanism can disengage the upper housing 154 allowing the port 100 to transition between the expanded configuration and the collapsed configuration. In an embodiment, the latching mechanism can be maintained in a locked position by the presence of the needle accessing the reservoir 101. For example, the needle accessing the reservoir 110 can engage the latching mechanism maintain the latching mechanism in the locked position, preventing the port 100 from transitioning between the expanded configuration and the collapsed configuration. Advantageously, the latching mechanism can prevent accidental transition of the port 100 between the expanded configuration and the collapsed configuration, for example, when the port 100 is in use, and/or when a needle is engaged with the reservoir 110.

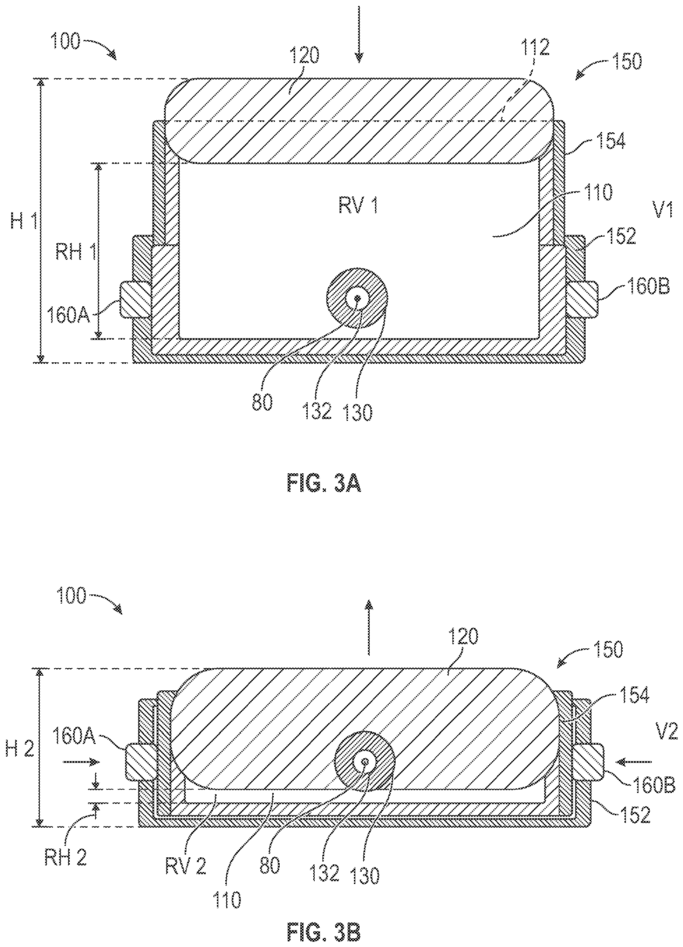

In an embodiment, as shown in FIGS. 3A-3B, the port 100 can include an actuator 160 coupled to the latching mechanism and configured to disengage the latching mechanism and/or transition the latching mechanism between the locked position and the unlocked position. In use, a clinician can transition the port 100 to the collapsed configuration by depressing the top surface of the port 100, as described herein (FIG. 3A). The latching mechanism can engage the upper housing 154 and retain the upper housing 154 in the collapsed configuration. The clinician can then actuate the actuator 160 to disengage the upper housing 154. The biasing member can then transition the upper housing to the expanded configuration (FIG. 3B).

In an embodiment, the latching mechanism can engage the upper housing 154 and maintain the upper housing 154 in the expanded configuration. In use, a clinician can actuate the actuator 160 to disengage the latching mechanism and allow the upper housing 154 to transition from the expanded configuration to the collapsed configuration. Advantageously, the actuator 160 and latching mechanism can be configured to prevent accidental transition of the port 100 from the expanded configuration to the collapsed configuration, for example, when the port 100 is in use and/or when a needle is engaged with the reservoir 110.

In an embodiment, the actuator 160 can be a push button, slider, rocker switch, or similar mechanical actuator disposed on an outer surface of the port 100, e.g. a top surface, side surface, etc. In an embodiment, the port 100 can include a first actuator 160A disposed on a first side surface of the port, and a second actuator 160B disposed on a second side surface of the port 100, opposite the first side surface. The clinician can actuate one of the first actuator 160A or the second actuator 160B by applying a lateral "pinching" force to the port 100. Advantageously, the lateral pinching force and can mitigate applying pressure directly to the patient.

In an embodiment, the actuator 160 can include a magnetic switch configured to be transitioned between a first position and a second position by the presence or absence of a magnetic field. In use, with the port 100 placed subcutaneously, a clinician can position a magnet (permanent magnet or electro-magnet) proximate the port 100, e.g. on a skin surface adjacent the port. The presence of the magnetic field can actuate the actuator 160 to disengage the latching mechanism and allow the port to transition between the expanded configuration and the collapsed configuration.

In an embodiment, the actuator 160 can be actuated by a signal from one or more modalities, e.g. magnetic, electro-magnetic, radio-frequency, acoustic, optical, or the like. For example, the actuator 160 can be actuated percutaneously by sending or receiving a signal to the port 100 from a device disposed external to the patient. The actuator 160 can disengage the latching mechanism and transition the port 100, as described herein.

Advantageously, the collapsed configuration can provide a smaller overall profile, size, or volume to the port 100. As such, the port 100 in the collapsed configuration can be disposed subcutaneously through a relatively smaller insertion site. Advantageously, the smaller insertion site can required fewer stitches, or no stitches, to close the insertion site leading to reduced scaring, improved recovery times, improved patient comfort and improved aesthetics. In an embodiment, the port 100 can be transitioned between the expanded configuration and the collapsed configuration by the clinician actuating the latching mechanism or actuator 160 percutaneously. When the port 100 has been disposed subcutaneously, the port 100 can be selectively transitioned from the collapsed (second) configuration to the expanded (first) configuration ready for use.

In an embodiment, the port 100 can be transitioned from the expanded configuration to the collapsed configuration outside of the patient, as described herein. Once placed subcutaneously, the port 100 can remain in the collapsed configuration. In use, a clinician can selectively transition the port 100 from the collapsed configuration to the expanded configuration ready for use. Once the treatment has been completed, the clinician can selectively transition the port 100 from the expanded configuration to the collapsed configuration. Advantageously, the port 100 can maintain the collapsed configuration after subcutaneous placement between uses providing a lower profile, reducing stretching of the skin, reducing scaring, and improving patient comfort and aesthetics. When the port 100 is required to be accessed the port 100 can be selectively transitioned to the expanded configuration by the clinician actuating the latching mechanism percutaneously.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A subcutaneous access port (100), comprising:
a body (150) including a lower housing (152) and an upper housing (154) slidably engaged therewith and co-operating to define a reservoir (110), the body (150) transitionable between an expanded configuration and a collapsed configuration, the collapsed configuration defining a smaller overall profile;
a needle penetrable septum (120) disposed in a reservoir opening (112) disposed in a top surface of the upper housing (154) and configured to provide percutaneous access thereto; and
a port stem (13) defining a stem lumen (132) in fluid communication with the reservoir (110),
wherein the reservoir (110) in the expanded configuration defines a first reservoir volume, and the reservoir (110) in the collapsed configuration defines a second reservoir volume, the second reservoir volume being less than the first reservoir volume.

2. The subcutaneous access port according to claim 1, wherein the port body (150) in the expanded configuration defines a first port height, and wherein the port body (150) in the collapsed configuration defines a second port height, the second port height being less than the first port height, and/or
wherein the port body (150) in the expanded configuration defines a first port volume, and the port body in the collapsed configuration defines a second port volume (150), the second port volume being less than the first port volume, and/or
wherein the reservoir (110) in the expanded configuration defines a first reservoir height, and the reservoir (110) in the collapsed configuration defines a second reservoir height, the second reservoir height being less than the first reservoir height.

3. The subcutaneous access port according to claim 1 or 2, wherein the second reservoir volume defines a zero volume or a *de minimis* volume.

4. The subcutaneous access port according to any one of claims 1-3, wherein the lower housing (152) is telescopically engaged with the upper housing (154) and slidably engaged to transition between the expanded configuration and the collapsed configuration, optionally
wherein an outer surface of the upper housing (154) is slidably engaged with an inner surface of the lower housing (152), or wherein an inner surface of the upper housing (154) is slidably engaged with an outer surface of the lower housing (152).

5. The subcutaneous access port according to any one of claims 1-4, wherein one of the upper housing (154) or the septum (120) occludes the stem lumen (132) when the port (150) is in the collapsed configuration, and/or
further including a biasing member configured to bias the port (150) towards the expanded configuration.

6. The subcutaneous access port according to any one of claims 1-5, further including a latching mechanism transitionable between a locked position and an unlocked position, and configured to retain the upper housing (154) in one of the expanded configuration or the collapsed configuration, optionally
wherein the latching mechanism is configured to lock the upper housing (154) in an expanded configuration when a needle accesses the reservoir (110).

7. The subcutaneous access port according to claim 6, wherein the latching mechanism is configured to engage and retain the upper housing (154) in a collapsed configuration and disengage the upper housing (154) when a top surface of the port (150) is depressed.

8. The subcutaneous access port according to any one of claims 6-7, further including an actuator (160) coupled to the latching mechanism and configured to transition the latching mechanism between a locked position and an unlocked position, optionally
wherein the actuator (160) is one of a push button, slider, or rocker switch disposed on one of a top surface or a side surface of the body, or wherein the actuator (160) includes a magnetic switch configured to transition the latching mechanism between a locked position and an unlocked position when a magnetic having a magnetic field is placed proximate the actuator or wherein the actuator (160) is configured to receive a signal to transition the latching mechanism between a locked position and an unlocked position, the signal including one of a magnetic, electro-magnetic, radio-frequency, acoustic, or optical modality signal.

9. A method of manufacturing a subcutaneous access port (100), comprising:
forming a port body (150) including a lower housing (152) and an upper housing (154) slidably engaged therewith and co-operating to define a reservoir (110), the body (150) transitionable between an expanded configuration and a collapsed configuration, the collapsed configuration defining a smaller overall profile;
coupling a needle penetrable septum (120) disposed in a reservoir opening (112) disposed in a top surface of the upper housing (154) and configured to provide percutaneous access thereto; and
forming a port stem (130) coupled to the body (150) and defining a stem lumen (132) in fluid communication with the reservoir (110),
wherein the reservoir (110) in the expanded configuration defines a first reservoir volume, and the reservoir (110) in the collapsed configuration defines a second reservoir volume, the second reservoir volume being less than the first reservoir volume.

10. The method according to claim 9, wherein the port body (150) in the expanded configuration defines a first port height, and wherein the port body (150) in the collapsed configuration defines a second port height, the second port height being less than the first port height, and/or
wherein the port body (150) in the expanded configuration defines a first port volume, and the port body (150) in the collapsed configuration defines a second port volume, the second port volume being less than the first port volume, and/or
wherein the reservoir (110) in the expanded configuration defines a first reservoir height, and the reservoir (110) in the collapsed configuration defines a second reservoir height, the second reservoir height being less than the first reservoir height.

11. The method according to claim 9 or 10, wherein the second reservoir volume defines a zero volume or a *de minimis* volume.

12. The method according to any one of claims 9-11, further including telescopically engaging the lower housing (152) with the upper housing (154) to slidably transition between the expanded configuration and the collapsed configuration, optionally
wherein an outer surface of the upper housing (154) is slidably engaged with an inner surface of the lower housing (152), further optionally
wherein an inner surface of the upper housing (154) is slidably engaged with an outer surface of the lower housing (152).

13. The method according to any one of claims 9-12, wherein one of the upper housing (153) or the septum (120) occludes the stem lumen (132) when the port (100) is in the collapsed configuration, and/or
further including forming a biasing member within the port body (150) configured to bias the port towards the expanded configuration.

14. The method according to any one of claims 9-13, further including a latching mechanism transitionable between a locked position and an unlocked position, and configured to retain the upper housing (154) in one of the expanded configuration or the collapsed configuration, optionally
wherein the latching mechanism is configured to lock the upper housing (154) in an expanded configuration when a needle accesses the reservoir (110), and/or
wherein the latching mechanism is configured to engage and retain the upper housing (154) in a collapsed configuration and disengage the upper housing (154) when a top surface of the port (150) is depressed.

15. The method according to claim 14, further including an actuator (160) coupled to the latching mechanism and configured to transition the latching mechanism between a locked position and an unlocked position, optionally
wherein the actuator (160) is one of a push button, slider, or rocker switch disposed on one of a top surface or a side surface of the body, or wherein the actuator (160) includes a magnetic switch configured to transition the latching mechanism between a locked position and an unlocked position when a magnetic having a magnetic field is placed proximate the actuator, or wherein the actuator (160) is configured to receive a signal to transition the latching mechanism between a locked position and an unlocked position, the signal including one of a magnetic, electro-magnetic, radio-frequency, acoustic, or optical modality signal.

## Patentansprüche

1. Subkutaner Zugangsport (100), umfassend:
einen Körper (150), der ein unteres Gehäuse (152) und ein damit verschiebbar in Eingriff stehendes oberes Gehäuse (154) einschließt, die zusammenwirken, um ein Reservoir (110) zu bilden, wobei der Körper (150) zwischen einer ausgefahrenen Konfiguration und einer eingefahrenen Konfiguration übergangsfähig ist, wobei die eingefahrene Konfiguration ein kleineres Gesamtprofil aufweist;
ein nadeldurchdringbares Septum (120), das in einer Reservoiröffnung (112) angeordnet ist, welche sich in einer Oberseite des oberen Gehäuses (154) befindet und ausgebildet ist, um einen perkutanen Zugang dorthin zu ermöglichen; und
einen Portstutzen (13), der ein Stutzenlumen (132) definiert, das in Fluidverbindung mit dem Reservoir (110) steht,
wobei das Reservoir (110) in der ausgefahrenen Konfiguration ein erstes Reservoirvolumen definiert und das Reservoir (110) in der eingefahrenen Konfiguration ein zweites Reservoirvolumen definiert, wobei das zweite Reservoirvolumen kleiner als das erste Reservoirvolumen ist.

2. Subkutaner Zugangsport nach Anspruch 1, wobei der Portkörper (150) in der ausgefahrenen Konfiguration eine erste Porthöhe definiert und wobei der Portkörper (150) in der eingefahrenen Konfiguration eine zweite Porthöhe definiert, wobei die zweite Porthöhe geringer ist als die erste Porthöhe, und/oder
wobei der Portkörper (150) in der ausgefahrenen Konfiguration ein erstes Portvolumen definiert und der Portkörper in der eingefahrenen Konfiguration ein zweites Portvolumen (150) definiert, wobei das zweite Portvolumen kleiner ist als das erste Portvolumen, und/oder
wobei das Reservoir (110) in der ausgefahrenen Konfiguration eine erste Reservoirhöhe definiert und das Reservoir (110) in der eingefahrenen Konfiguration eine zweite Reservoirhöhe definiert, wobei die zweite Reservoirhöhe geringer ist als die erste Reservoirhöhe.

3. Subkutaner Zugangsport nach Anspruch 1 oder 2, wobei das Volumen des zweiten Reservoirs ein Nullvolumen oder ein *de minimis* Volumen definiert.

4. Subkutaner Zugangsport nach einem der Ansprüche 1-3, wobei das untere Gehäuse (152) teleskopartig mit dem oberen Gehäuse (154) in Eingriff gebracht ist und verschiebbar gelagert ist, um zwischen der ausgefahrenen und der eingefahrenen Konfiguration überzugehen, wahlweise
wobei eine Außenfläche des oberen Gehäuses (154) gleitend mit einer Innenfläche des unteren Gehäuses (152) in Eingriff steht, oder wobei eine Innenfläche des oberen Gehäuses (154) gleitend mit einer Außenfläche des unteren Gehäuses (152) in Eingriff steht.

5. Subkutaner Zugangsport nach einem der Ansprüche 1-4, wobei entweder das obere Gehäuse (154) oder das Septum (120) das Schaftlumen (132) verschließt, wenn sich der Port (150) in der eingefahrenen Konfiguration befindet, und/oder
weiter einschließend ein Vorspannelement, das ausgebildet ist, um den Port (150) in Richtung der ausgefahrenen Konfiguration vorzuspannen.

6. Subkutaner Zugangsport nach einem der Ansprüche 1-5, weiter einschließend einen Verriegelungsmechanismus, der zwischen einer verriegelten Position und einer entriegelten Position übergangsfähig ist und ausgebildet ist, um das obere Gehäuse (154) entweder in der ausgefahrenen oder in der eingefahrenen Konfiguration zu halten, wahlweise
wobei der Verriegelungsmechanismus ausgebildet ist, um das obere Gehäuse (154) in einer ausgefahrenen Konfiguration zu arretieren, wenn eine Nadel in das Reservoir (110) eingeführt wird.

7. Subkutaner Zugangsport nach Anspruch 6, wobei der Verriegelungsmechanismus ausgebildet ist, um das obere Gehäuse (154) in einer eingefahrenen Konfiguration in Eingriff zu bringen und festzuhalten und das obere Gehäuse (154) freizugeben, wenn eine Oberseite des Ports (150) eingedrückt ist.

8. Subkutaner Zugangsport nach einem der Ansprüche 6-7, weiter einschließend einen Aktuator (160), der mit dem Verriegelungsmechanismus verbunden und ausgebildet ist, dass er den Verriegelungsmechanismus zwischen einer verriegelten Position und einer entriegelten Position überführt, wahlweise
wobei der Aktuator (160) entweder ein Druckknopf, ein Schieberegler oder ein Wippschalter ist, der an der Oberseite oder an einer Seitenfläche des Körpers angeordnet ist, oder wobei der Aktuator (160) einen Magnetschalter einschließt, der ausgebildet ist, dass er den Verriegelungsmechanismus zwischen einer verriegelten Position und einer entriegelten Position überführt, wenn ein Magnet mit einem Magnetfeld in die Nähe des Aktuators gebracht wird, oder wobei der Aktuator (160) ausgebildet ist, um ein Signal zu empfangen, um den Verriegelungsmechanismus zwischen einer verriegelten Position und einer entriegelten Position überzuführen, wobei das Signal ein Signal in Form einer magnetischen, elektromagnetischen, hochfrequenten, akustischen oder optischen Modalität einschließt.

9. Verfahren zum Herstellen eines subkutanen Zugangsports (100), umfassend:
Bilden eines Portkörpers (150), der ein unteres Gehäuse (152) und ein damit verschiebbar in Eingriff stehendes oberes Gehäuse (154) einschließt, die zusammenwirken, um ein Reservoir (110) zu bilden, wobei der Körper (150) zwischen einer ausgefahrenen Konfiguration und einer eingefahrenen Konfiguration übergangsfähig ist, wobei die eingefahrene Konfiguration ein kleineres Gesamtprofil aufweist;
Koppeln eines mit einer Nadel durchstechbaren Septums (120), das in einer Reservoiröffnung (112) angeordnet ist, welche sich in einer Oberseite des oberen Gehäuses (154) befindet und ausgebildet ist, um einen perkutanen Zugang dorthin zu ermöglichen; und
Bilden eines Portstutzens (130), der mit dem Körper (150) gekoppelt ist und ein Stutzenlumen (132) definiert, das in Fluidverbindung mit dem Reservoir (110) steht,
wobei das Reservoir (110) in der ausgefahrenen Konfiguration ein erstes Reservoirvolumen definiert und das Reservoir (110) in der eingefahrenen Konfiguration ein zweites Reservoirvolumen definiert, wobei das zweite Reservoirvolumen kleiner als das erste Reservoirvolumen ist.

10. Verfahren nach Anspruch 9, wobei der Portkörper (150) in der ausgefahrenen Konfiguration eine erste Porthöhe definiert und wobei der Portkörper (150) in der eingefahrenen Konfiguration eine zweite Porthöhe definiert, wobei die zweite Porthöhe geringer ist als die erste Porthöhe, und/oder
wobei der Portkörper (150) in der ausgefahrenen Konfiguration ein erstes Portvolumen definiert und der Portkörper (150) in der eingefahrenen Konfiguration ein zweites Portvolumen definiert, wobei das zweite Portvolumen kleiner ist als das erste Portvolumen, und/oder
wobei das Reservoir (110) in der ausgefahrenen Konfiguration eine erste Reservoirhöhe definiert und das Reservoir (110) in der eingefahrenen Konfiguration eine zweite Reservoirhöhe definiert, wobei die zweite Reservoirhöhe geringer ist als die erste Reservoirhöhe.

11. Verfahren nach Anspruch 9 oder 10, wobei das Volumen des zweiten Reservoirs ein Nullvolumen oder ein *de minimis* Volumen definiert.

12. Verfahren nach einem der Ansprüche 9-11, weiter einschließend das untere Gehäuse (152) teleskopartig mit dem oberen Gehäuse (154) in Eingriff bringen, um einen gleitenden Übergang zwischen der ausgefahrenen und der eingefahrenen Konfiguration zu ermöglichen, wahlweise
wobei eine Außenfläche des oberen Gehäuses (154) gleitend mit einer Innenfläche des unteren Gehäuses (152) in Eingriff steht, weiter wahlweise
wobei eine Innenfläche des oberen Gehäuses (154) gleitend mit einer Außenfläche des unteren Gehäuses (152) in Eingriff steht.

13. Verfahren nach einem der Ansprüche 9-12, wobei entweder das obere Gehäuse (153) oder das Septum (120) das Schaftlumen (132) verschließt, wenn sich der Port (100) in der eingefahrenen Konfiguration befindet, und/oder
weiter einschließend das Bilden eines Vorspannelements innerhalb des Portkörpers (150), das ausgebildet ist, um den Port in Richtung der expandierten Konfiguration vorzuspannen.

14. Verfahren nach einem der Ansprüche 9-13, weiter einschließend einen Verriegelungsmechanismus, der zwischen einer verriegelten Position und einer entriegelten Position übergangsfähig ist und ausgebildet ist, um das obere Gehäuse (154) entweder in der ausgefahrenen oder in der eingefahrenen Konfiguration zu halten, wahlweise
wobei der Verriegelungsmechanismus ausgebildet ist, um das obere Gehäuse (154) in einer ausgefahrenen Konfiguration zu arretieren, wenn eine Nadel in das Reservoir (110) eingeführt wird, und/oder
wobei der Verriegelungsmechanismus ausgebildet ist, um das obere Gehäuse (154) in einer eingefahrenen Konfiguration in Eingriff zu bringen und festzuhalten und das obere Gehäuse (154) freizugeben, wenn eine Oberseite des Ports (150) eingedrückt ist.

15. Verfahren nach Anspruch 14, weiter einschließend einen Aktuator (160), der mit dem Verriegelungsmechanismus verbunden und ausgebildet ist, dass er den Verriegelungsmechanismus zwischen einer verriegelten Position und einer entriegelten Position überführt, wahlweise
wobei der Aktuator (160) entweder ein Druckknopf, ein Schieberegler oder ein Wippschalter ist, der an der Oberseite oder einer Seitenfläche des Körpers angeordnet ist, oder wobei der Aktuator (160) einen Magnetschalter einschließt, der ausgebildet ist, dass er den Verriegelungsmechanismus zwischen einer verriegelten Position und einer entriegelten Position überführt, wenn ein Magnet mit einem Magnetfeld in die Nähe des Aktuators gebracht wird, oder wobei der Aktuator (160) ausgebildet ist, um ein Signal zu empfangen, um den Verriegelungsmechanismus zwischen einer verriegelten Position und einer entriegelten Position überzuführen, wobei das Signal ein Signal in Form einer magnetischen, elektromagnetischen, hochfrequenten, akustischen oder optischen Modalität einschließt.

## Revendications

1. Cathéter (100) d'accès sous-cutané, comprenant :
un corps (150) incluant un boîtier inférieur (152) et un boîtier supérieur (154) mis en prise de manière coulissante avec celui-ci et coopérant pour délimiter un réservoir (110), le corps (150) étant permutable entre une configuration dépliée et une configuration repliée, la configuration repliée délimitant un profil global plus réduit ;
un septum (120) pénétrable par aiguille disposé dans une ouverture (112) de réservoir disposée dans une surface supérieure du boîtier supérieur (154) et configuré pour fournir un accès percutané à celui-ci ; et
un tube (13) de cathéter délimitant une lumière (132) de tube en communication fluidique avec le réservoir (110),
dans lequel le réservoir (110) dans la configuration dépliée délimite un premier volume de réservoir, et le réservoir (110) dans la configuration repliée délimite un deuxième volume de réservoir, le deuxième volume de réservoir étant inférieur au premier volume de réservoir.

2. Cathéter d'accès sous-cutané selon la revendication 1, dans lequel le corps (150) de cathéter dans la configuration dépliée délimite une première hauteur de cathéter, et dans lequel le corps (150) de cathéter dans la configuration repliée délimite une deuxième hauteur de cathéter, la deuxième hauteur de cathéter étant inférieure à la première hauteur de cathéter, et/ou
dans lequel le corps (150) de cathéter dans la configuration dépliée délimite un premier volume de cathéter, et le corps de cathéter dans la configuration repliée délimite un deuxième volume de cathéter (150), le deuxième volume de cathéter étant inférieur au premier volume de cathéter, et/ou
dans lequel le réservoir (110) dans la configuration dépliée délimite une première hauteur de réservoir, et le réservoir (110) dans la configuration repliée délimite une deuxième hauteur de réservoir, la deuxième hauteur de réservoir étant inférieure à la première hauteur de réservoir.

3. Cathéter d'accès sous-cutané selon la revendication 1 ou la revendication 2, dans lequel le deuxième volume de réservoir délimite un volume nul ou un volume *de minimis.*

4. Cathéter d'accès sous-cutané selon l'une quelconque des revendications 1 à 3, dans lequel le boîtier inférieur (152) est mis en prise de manière télescopique avec le boîtier supérieur (154) et mis en prise de manière coulissante pour permuter entre la configuration dépliée et la configuration repliée, facultativement
dans lequel une surface externe du boîtier supérieur (154) est mise en prise de manière coulissante avec une surface interne du boîtier inférieur (152), ou dans lequel une surface interne du boîtier supérieur (154) est mise en prise de manière coulissante avec une surface externe du boîtier inférieur (152).

5. Cathéter d'accès sous-cutané selon l'une quelconque des revendications 1 à 4, dans lequel l'un parmi le boîtier supérieur (154) ou le septum (120) obture la lumière (132) de tube lorsque le cathéter (150) est dans la configuration repliée, et/ou
incluant en outre un organe de sollicitation configuré pour solliciter le cathéter (150) vers la configuration dépliée.

6. Cathéter d'accès sous-cutané selon l'une quelconque des revendications 1 à 5, incluant en outre un mécanisme de verrouillage permutable entre une position bloquée et une position non bloquée, et configuré pour retenir le boîtier supérieur (154) dans l'une parmi la configuration dépliée ou la configuration repliée, facultativement
dans lequel le mécanisme de verrouillage est configuré pour verrouiller le boîtier supérieur (154) dans une configuration dépliée lorsqu'une aiguille accède au réservoir (110).

7. Cathéter d'accès sous-cutané selon la revendication 6, dans lequel le mécanisme de verrouillage est configuré pour mettre en prise et retenir le boîtier supérieur (154) dans une configuration repliée et désolidariser le boîtier supérieur (154) lorsqu'une surface supérieure du cathéter (150) est enfoncée.

8. Cathéter d'accès sous-cutané selon l'une quelconque des revendications 6 à 7, incluant en outre un actionneur (160) accouplé au mécanisme de verrouillage et configuré pour permuter le mécanisme de verrouillage entre une position bloquée et une position non bloquée, facultativement
dans lequel l'actionneur (160) est l'un parmi un bouton-poussoir, un coulisseau ou un interrupteur à culbuteur disposé sur l'une parmi une surface supérieure ou une surface latérale du corps, ou dans lequel l'actionneur (160) inclut un interrupteur magnétique configuré pour permuter le mécanisme de verrouillage entre une position bloquée et une position non bloquée lorsqu'un aimant présentant un champ magnétique est placé à proximité de l'actionneur ou dans lequel l'actionneur (160) est configuré pour recevoir un signal pour permuter le mécanisme de verrouillage entre une position bloquée et une position non bloquée, le signal incluant l'un parmi un signal de modalité magnétique, électro-magnétique, radio-fréquence, acoustique ou optique.

9. Procédé de fabrication d'un cathéter (100) d'accès sous-cutané, comprenant :
le formage d'un corps (150) de cathéter incluant un boîtier inférieur (152) et un boîtier supérieur (154) mis en prise de manière coulissante avec celui-ci et coopérant pour délimiter un réservoir (110), le corps (150) étant permutable entre une configuration dépliée et une configuration repliée, la configuration repliée délimitant un profil global plus réduit ;
l'accouplement d'un septum (120) pénétrable par aiguille disposé dans une ouverture (112) de réservoir disposée dans une surface supérieure du boîtier supérieur (154) et configuré pour fournir un accès percutané à celui-ci ; et
le formage d'un tube (130) de cathéter accouplé au corps (150) et délimitant une lumière (132) de tube en communication fluidique avec le réservoir (110),
dans lequel le réservoir (110) dans la configuration dépliée délimite un premier volume de réservoir, et le réservoir (110) dans la configuration repliée délimite un deuxième volume de réservoir, le deuxième volume de réservoir étant inférieur au premier volume de réservoir.

10. Procédé selon la revendication 9, dans lequel le corps (150) de cathéter dans la configuration dépliée délimite une première hauteur de cathéter, et dans lequel le corps (150) de cathéter dans la configuration repliée délimite une deuxième hauteur de cathéter, la deuxième hauteur de cathéter étant inférieure à la première hauteur de cathéter, et/ou
dans lequel le corps (150) de cathéter dans la configuration dépliée délimite un premier volume de cathéter, et le corps (150) de cathéter dans la configuration repliée délimite un deuxième volume de cathéter, le deuxième volume de cathéter étant inférieur au premier volume de cathéter, et/ou
dans lequel le réservoir (110) dans la configuration dépliée délimite une première hauteur de réservoir, et le réservoir (110) dans la configuration repliée délimite une deuxième hauteur de réservoir, la deuxième hauteur de réservoir étant inférieure à la première hauteur de réservoir.

11. Procédé selon la revendication 9 ou la revendication 10, dans lequel le deuxième volume de réservoir délimite un volume nul ou un volume *de minimis.*

12. Procédé selon l'une quelconque des revendications 9 à 11, incluant en outre la mise en prise télescopique du boîtier inférieur (152) avec le boîtier supérieur (154) pour permuter de manière coulissante entre la configuration dépliée à la configuration repliée, facultativement
dans lequel une surface externe du boîtier supérieur (154) est mise en prise de manière coulissante avec une surface interne du boîtier inférieur (152), encore facultativement
dans lequel une surface interne du boîtier supérieur (154) est mise en prise de manière coulissante avec une surface externe du boîtier inférieur (152).

13. Procédé selon l'une quelconque des revendications 9 à 12, dans lequel l'un parmi le boîtier supérieur (153) ou le septum (120) obture la lumière (132) de tube lorsque le cathéter (100) est dans la configuration repliée, et/ou
incluant en outre le formage d'un organe de sollicitation à l'intérieur du corps (150) de cathéter configuré pour solliciter le cathéter vers la configuration dépliée.

14. Procédé selon l'une quelconque des revendications 9 à 13, incluant en outre un mécanisme de verrouillage permutable entre une position bloquée et une position non bloquée, et configuré pour retenir le boîtier supérieur (154) dans l'une parmi la configuration dépliée ou la configuration repliée, facultativement
dans lequel le mécanisme de verrouillage est configuré pour verrouiller le boîtier supérieur (154) dans une configuration dépliée lorsqu'une aiguille accède au réservoir (110), et/ou
dans lequel le mécanisme de verrouillage est configuré pour mettre en prise et retenir le boîtier supérieur (154) dans une configuration repliée et désolidariser le boîtier supérieur (154) lorsqu'une surface supérieure du cathéter (150) est enfoncée.

15. Procédé selon la revendication 14, incluant en outre un actionneur (160) accouplé au mécanisme de verrouillage et configuré pour permuter le mécanisme de verrouillage entre une position bloquée et une position non bloquée, facultativement
dans lequel l'actionneur (160) est l'un parmi un bouton-poussoir, un coulisseau ou un interrupteur à culbuteur disposé sur l'une parmi une surface supérieure ou une surface latérale du corps, ou dans lequel l'actionneur (160) inclut un interrupteur magnétique configuré pour permuter le mécanisme de verrouillage entre une position bloquée et une position non bloquée lorsqu'un aimant présentant un champ magnétique est placé à proximité de l'actionneur, ou dans lequel l'actionneur (160) est configuré pour recevoir un signal pour permuter le mécanisme de verrouillage entre une position bloquée et une position non bloquée, le signal incluant l'un parmi un signal de modalité magnétique, électro-magnétique, radio-fréquence, acoustique ou optique.
